**Europäisches Patentamt**

**European Patent Office**

**Office européen des brevets**

(19)

(11) Veröffentlichungsnummer: **0 224 146 B1**

# EUROPÄISCHE PATENTSCHRIFT

(12)

(45) Veröffentlichungstag der Patentschrift: **29.04.92**

(51) Int. Cl.⁵: **A61K 9/36, A61K 9/22**

(21) Anmeldenummer: **86115836.8**

(22) Anmeldetag: **14.11.86**

(54) Pharmazeutisches Präparat zur kontrollierten Freisetzung von Wirkstoffen.

(30) Priorität: **18.11.85 US 799229**

(43) Veröffentlichungstag der Anmeldung:
**03.06.87 Patentblatt 87/23**

(45) Bekanntmachung des Hinweises auf die
Patenterteilung:
**29.04.92 Patentblatt 92/18**

(84) Benannte Vertragsstaaten:
**BE CH DE FR GB IT LI LU NL SE**

(56) Entgegenhaltungen:
EP-A- 0 066 505
EP-A- 0 158 277
WO-A-84/04674
FR-A- 1 290 661
US-A- 4 140 756

(73) Patentinhaber: **F. HOFFMANN-LA ROCHE AG**
**Postfach 3255**
**CH-4002 Basel(CH)**

(72) Erfinder: **Shah, Navnit Hargovindas**
**203 Beverly Hill Road**
**Clifton, N.J.(US)**

(74) Vertreter: **Lederer, Franz, Dr. et al**
**Patentanwalt Dr. Franz Lederer Lucile-**
**Grahn-Strasse 22**
**W-8000 München 80(DE)**

## Beschreibung

Die vorliegende Erfindung betrifft ein pharmazeutisches Präparat zur kontrollierten Freisetzung von Wirkstoffen, das gekennzeichnet ist durch einen Tablettenkern, der einen wasserlöslichen Wirkstoff in einer wasserunlöslichen polymeren Matrix enthält, die mit einem die Freisetzungsgeschwindigkeit kontrollierenden Ueberzug versehen ist. Die Erfindung betrifft weiterhin ein Verfahrens zur Herstellung solcher Präparate. Das erfindungsgemässe Präparat setzt den Wirkstoff durch die Membran, die die unlösliche polymere Matrix überzieht, mit einer niedrigen und gleichmässigen Geschwindigkeit frei.

Das Dokument US-A-4 140 756 (Gallian) beschreibt Präparate , die einen Kern aus einem Material enthalten, das eine wachsartige Konsistenz aufweist. Solche Materialien bleiben im Magen nicht formstabil, sie brechen auf und werden emulgiert.

Insbesondere betrifft die Erfindung ein pharmazeutisches Präparat zur kontrollierten Freisetzung von Wirkstoffen in Tablettenform, welches dadurch gekennzeichnet ist, dass es

(1) einen Kern enthält, der 90-95 Gew.-% der Tablette ausmacht und der zu

(a) 65-95 Gew.-% aus einem wasserlöslichen Wirkstoff und zu

(b) 5-35 Gew.-% aus Aethylcellulose besteht und

(2) einen Membranüberzug aufweist, der 5-10 Gew.-% der Tablette ausmacht und der aus einem die Freisetzungsgeschwindigkeit kontrollierenden Polymer besteht.

Die unlösliche polymere Matrix des Tablettenkernes soll hinreichend fest sein, die Freisetzungsgeschwindigkeit des Wirkstoffes aus dem Kern zu verzögern. Darüber hinaus vermittelt der Tablettenkern ein Gerüst für die äussere, die Freisetzungsgeschwindigkeit kontrollierende Membran. Der polymere Matrixkern verhindert ein Schrumpfen und Reissen des Membranüberzuges, wenn dieser gastrointestinalen Flüssigkeiten ausgesetzt wird. Er erschwert auch das Eindringen gastrointestinaler Flüssigkeit, wodurch der Wirkstoff in der Matrix für einen längeren Zeitraum festgehalten wird.

Für den erfindungsgemässen Matrixkern ist Aethylcellulose mit einem Viskositätsbereich von etwa 3-100 mPas (cP) und einem Aethoxygehalt von etwa 45-49,5% bevorzugt.

Zusätzlich zu dem polymeren Material kann der Matrixkern gewünschtenfalls ein öl- oder wachsartiges Material wie Weichmacher enthalten, wodurch die Freisetzungsgeschwindigkeiten des Präparates verändert werden können. Ein Zusatz von Wachs erlaubt es auch höhere Konzentrationen an Wirkstoff in das Präparat einzuarbeiten. Geeignete öl- oder wachsartige Stoffe sind Fettsäuren wie Stearinsäure, langkettige Fettalkohole wie Stearylalkohol, Cetylalkohol, Carnaubawachs, Bienenwachs und weisses Wachs. Geeignete Oele sind Glyceride von $C_6$-$C_{18}$ -Fettsäuren, Mineralöl, Triacetin, Dibutylphthalat, Dibutylsebacat und Triäthylcitrat.

Der polymere Matrixkern kann weiterhin andere pharmazeutisch annehmbare Excipientien wie Bindemittel, Füllstoffe, Stabilisatoren, Kompressionshilfen, Schmiermittel, Granulationshilfen, Fliessmittel und ähnliches enthalten. Es ist möglich, mit der Wahl inerter pharmazeutischer Excipientien die Freisetzungsgeschwindigkeit des Präparates zu modifizieren. Beispielsweise erhöhen lösliche Excipientien, beispielsweise Lactose, Sucrose, Mannit, Dextrose, Sorbit und ähnliches, die Freisetzungsrate des Wirkstoffs, während unlösliche Excipientien, beispielsweise Calciumsulfat, Bariumsulfat, Mono-, Di- oder Tri-basisches Calciumphosphat und ähnliches die Freisetzungsrate aus dem Kern vermindern.

Der Membranüberzug besteht aus einem oder mehreren Film-bildenden Materialien, die die Freisetzungsrate des Wirkstoffs aus der Formulierung kontrollieren können. Beispiele von Polymeren, die erfindungsgemäss als Membranüberzüge in Betracht kommen, sind Aethylcellulose und Gemische von Aethylcellulose und Hydroxypropylcellulose oder Hydroxypropylmethylcellulose. Ein bevorzugter Membranüberzug ist Aethylcellulose mit einem Viskositätsbereich von etwa 3-100 mPas (cP) und einem Aethoxygehalt von etwa 45-49,5% und Hydroxypropylmethylcellulose mit einem Methoxygehalt von 16,5-32%, einem Hydroxypropylgehalt von etwa 4-33%, einem Methoxylierungsgrad zwischen 1,12 und 2,03 und einem Viskositätsbereich von etwa 3 bis 100,000 mPas (cP).

Um die Gefahr des Härtens und Reissens des Membranüberzuges zu vermindern ist es oft wünschenswert, einen Weichmacher in Kombination mit dem polymeren Ueberzugsmaterial zu verwenden. Beispiele von Weichmachern die erfindungsgemäss angewandt werden können sind Triacetin, Propylenglykol, Polyäthylenglykol mit einem Molekulargewicht von 200 bis 1000, Dibutylphthalat, Dibutylsebacat, Triäthylcitrat, Fettsäuren und Fettsäureglyceride von $C_6$-$C_{18}$--Fettsäuren und ähnliches. Der Membranüberzug kann weiterhin andere Excipientien für Ueberzüge enthalten, wie Opakisierungsmittel, Pigmente und Farbstoffe.

Die Präparate können erfindungsgemäss dadurch hergestellt werden, dass man einen wie oben definierten Tablettenkern mit einem wie oben definierten Ueberzug versieht.

Die erfindungsgemässen Präparate können unter Anwendung an sich bekannter Technologien hergestellt werden. In den meisten Fällen ist es bei der Bildung des Kerns notwendig, die Technologie der Feuchtgranulierung anzuwenden. Falls es die physikalischen Eigenschaften der Inhaltsstoffe erlauben, können die Tabletten durch direkte Verpressung eines homogenen Gemischs der Inhaltsstoffe hergestellt werden. Der erfindungsgemäss verwendete Kern kann auf konventionellen Tablettierapparaturen hergestellt werden.

Das Aufbringen des Membranüberzugs auf den Matrixkern kann mittels an sich bekannter Technologien, z.B. in Dragierkesseln oder Wirbelschichtgeräten erfolgen.

Die erfindungsgemässen Präparate eignen sich für wasserlösliche Wirkstoffe, insbesondere für Kaliumchlorid. Weitere Beispiele von geeigneten Wirkstoffen sind Cibenzolinsuccinat, Pseudoephedrinhydrochlorid, Chorzitronensäure, Procainamid und Theophyllin.

Die erfindungsgemässen Präparate gewährleisten eine Freisetzung des im Kern enthaltenen Wirkstoffe, die im wesentlichen nullter Ordnung ist.

Die nachstehenden Beispiele erläutern die Erfindung.

Beispiele 1

Ein Präparat zur kontrollierten Freisetzung von Kaliumchlorid wurde wie folgt hergestellt:

| Inhaltsstoff | mg/Tablette |
|---|---|
| Kaliumchlorid | 600 |
| Aethylcellulose | 100 |
| Neobee M5 (Triglycerid mittlerer Kettenlänge) | 35 |
| Magnesiumstearat | 3 |
| | 738 mg |

Die Aethylcellulose und Neobee M5 wurden in 10% ihres Gewichtes Aethanol gelöst und nach Zusatz von Kaliumchlorid granuliert. Das Granulationsgemisch wurde bei 55°C auf einen Feuchtigkeitsgehalt von weniger als 2% getrocknet. Das Gemisch wurde gesiebt, mit dem Magnesiumstearat vermischt und danach zu Tabletten verpresst.

Der Tablettenkern wurde mit einer polymeren Membran folgender Zusammensetzung überzogen:

| Inhaltsstoffe | |
|---|---|
| Aethylcellulose 50 mPas (cP) | 3 |
| Hydroxypropylmethylcellulose | 2 |
| Stearinsäure | 1 |
| Methylenchlorid | 47,5 |
| Alkohol | 47,5 |
| | 100,0 |

Der Filmüberzug wurde so aufgebracht, dass er 5-8% des Tablettenendgewichtes betrug.

Die Freisetzungscharakteristiken sind aus Tabelle I ersichtlich.

Beispiel 2

Ein Präparat für die kontrolliert Freisetzung von Kaliumchlorid wurde wie folgt hergestellt:

| Inhaltsstoffe | |
|---|---|
| Kaliumchlorid | 600 |
| Ethocel (micromesh) | 80 |
| Ethocel | 20 |
| Stearinsäure | 5 |
| Magnesiumstearat | 3 |
| | 703 mg |

Das Kaliumchlorid und Ethocel (micromesh) wurden gründlich gemischt und mit Ethocel und Stearinsäure in 10% ihres Gewichtes Aethanol granuliert. Das Granulat wurde über Nacht bei 55°C getrocknet, gesiebt, mit Stearinsäure und Magnesiumstearat gemischt und zu Tabletten verpresst.

Der Tablettenkern wurde mit einer polymeren Membran der folgenden Zusammensetzung überzogen:

| Inhaltsstoffe | Gewichtsprozent |
|---|---|
| Ethocel 10 mPas (cP) | 3 |
| Methylcellulose E15 | 2 |
| Dibutylsebacat | 1 |
| Methylenchlorid | 47,5 |
| Alkohol | 47,5 |
| | 100,0 |

Der Ueberzug wurde so aufgebracht, dass das Gewicht 5-8% des Tablettenendgewichts betrug.
Die Freisetzungscharakteristiken sind in Tabelle I aufgeführt.

Beispiel 3

Ein Präparat zur kontrollierten Freisetzung von Kaliumchlorid wurde wie folgt hergestellt:

| Inhaltsstoffe | mg/Tablette |
|---|---|
| Kaliumchlorid | 1500 |
| Ethocel 50 mPas (cP) | 80 |
| Stearinsäure | 40 |
| Magnesiumstearat | 3 |

Kaliumchlorid und ein Teil des Ethocels und der Stearinsäure wurden gründlich gemischt und mit einer Lösung des restlichen Ethocels und der Stearinsäure granuliert. Das Granulat wurde bei 55°C getrocknet und gesiebt. Danach wurde Magnesiumstearat zugesetzt und das Präparat zu Tabletten verpresst. Die gepressten Tabletten wurden 2 Stunden auf 76°C erhitzt.

Der Tablettenkern wurde mit einer polymeren Membran der folgenden Zusammesetzung überzogen:

| Inhaltsstoffe | Gewichtsprozent |
|---|---|
| Ethocel 50 mPas (cP) | 3 |
| Methocel E 15 | 2 |
| Neobee M5 (Triglycerid mittlerer Kettenlänge) | 1 |
| Methylenchlorid | 47,5 |
| Alkohol | 47,5 |
| | 100,0 |

Der Ueberzug wurde bis zu einem Gewicht von 5% des Tablettenendgewichtes aufgebracht.
Die Freisetzungscharakteristiken sind in Tabelle I aufgeführt.

4

Beispiel 4

Ein Präparat mit verzögerter Freisetzung von Kaliumchlorid wurde wie folgt hergestellt:

| Inhaltsstoffe | |
|---|---|
| Kaliumchlorid | 600 |
| Aethylcellulose 50 mPas (cP) | 50 |
| Dibutylsebacat | 15 |
| Stearinsäure | 5 |
| Magnesiumstearat | 3 |

Das mikronisierte Kaliumchlorid wurde mit einer Lösung von Ethocel und Dibutylsebacat in 10% ihres Gewichtes Aethanol granuliert. Das getrocknete Material wurde gesiebt. Stearinsäure und Magnesiumstearat wurden zugesetzt und die Mischung zu Tabletten verpresst.

Der Tablettenkern wurde mit einer polymeren Membran der folgenden Zusammensetzung überzogen:

| Inhaltsstoffe | Gewichtsprozent |
|---|---|
| Ethocel 50 mPas (cP) | 3 |
| Methocel E 5 | 1 |
| Neobee M5 | 1 |
| Methylenchlorid | 47,5 |
| Magnesiumstearat | 47,5 |
| | 100,0 |

Der Ueberzug wurde bis zu einem Gewicht von etwa 5-8% des Tablettenendgewichts aufgebracht.

Die Freisetzungscharakteristiken sind in Tabelle I angeführt.

## Tabelle I

Freisetzungsraten von Kaliumchlorid in Wasser bestimmt nach der USP-Korb-Methode/100 U/min

% Freisetzung nach

| Präparat von | Ueberzugsstärke [% des Tabletten-gewichts] | 1h | 2h | 3h | 4h | 5h | 6h | 7h | 8h | 9h |
|---|---|---|---|---|---|---|---|---|---|---|
| Beispiel 1 | 5 | 15 | 31 | 44 | 61 | 74 | 87 | 96 | – | – |
|  | 8 | 11 | 21 | 32 | 43 | 54 | 65 | 75 | 87 | 94 |
| Beispiel 2 | 5 | 14 | 28 | 41 | 55 | 72 | 82 | 91 | 95 | – |
|  | 8 | 10 | 21 | 30 | 41 | 51 | 60 | 71 | 82 | 89 |
| Beispiel 3* | 5 | 8 | 16 | 25 | 32 | 41 | 49 | 56 | 66 | 74 |
| Beispiel 4 | 5 | 9 | 18 | 26 | 35 | 45 | 55 | 64 | 74 | 82 |

* Freisetzungsgeschwindigkeit wurde bei verschiedenen pH bestimmt und erwies sich als unabhängig vom pH

**Patentansprüche**

1. Pharmazeutisches Präparat in Tablettenform, dadurch gekennzeichnet, dass es

(1) einen Kern enthält, der 90-95 Gew.-% der Tablette ausmacht und der zu

    (a) 65-95 Gew.-% aus einem wasserlöslichen Wirkstoff und zu

    (b) 5-35 Gew.-% aus Aethylcellulose besteht und

(2) einen Membranüberzug aufweist, der 5-10 Gew.-% der Tablette ausmacht und der aus einem die Freisetzungsgeschwindigkeit kontrollierenden Polymer besteht.

**2.** Präparat gemäss Anspruch 1, dadurch gekennzeichnet, dass das Polymer ein Aethylcellulosepolymer mit einem Viskositätsbereich von 3-100 mPas (cP) und einem Aethoxygehalt von 45-49,5% ist.

**3.** Präparat gemäss Anspruch 1, dadurch gekennzeichnet, dass die wasserunlösliche polymere Matrix weiterhin ein öl- oder wachsartiges Material enthält.

**4.** Präparat gemäss Anspruch 3, dadurch gekennzeichnet, dass das öl- oder wachsartige Material Stearinsäure, Stearylalkohol, Cetylalkohol, Carnaubawachs, Bienenwachs, weisses Wachs oder ein Glycerid von $C_6$-$C_{18}$-Fettsäurenist.

**5.** Präparat gemäss Anspruch 4, dadurch gekennzeichnet, dass der Membranüberzug aus Aethylcellulose oder Gemischen von Aethylcellulose und Hydroxypropylmethylcellulose oder Hydroxypropylcellulose besteht.

**6.** Präparat gemäss Anspruch 5, dadurch gekennzeichnet, dass der Membranüberzug aus Aethylcellulose besteht.

**7.** Präparat gemäss Anspruch 5, dadurch gekennzeichnet, dass der Membranüberzug weiterhin einen Weichmacher enthält.

**8.** Präparat gemäss Anspruch 5, dadurch gekennzeichnet, dass der Weichmacher Triacetin, Propylenglykol, Polyäthylenglykol mit einem Molekulargewicht von 200 bis 1000, Dibutylphthalat, Dibutylsebacat, Triäthylcitrat, Fettsäuren oder ein Glycerid von $C_6$-$C_{18}$-Fettsäuren ist.

**9.** Präparat gemäss Anspruch 8, dadurch gekennzeichnet, dass der Weichmacher ein Glycerid von $C_6$-$C_{18}$-Fettsäuren ist.

## Claims

**1.** A pharmaceutical preparation in tablet form, characterized in that it

(1) contains a core which constitutes 90-95 wt.% of the tablet and which comprises

    (a) 65-95 wt.% of a water-soluble active ingredient and

    (b) 5-35 wt.% of ethyl cellulose and

(2) has a membrane coating which constitutes 5-10 wt.% of the tablet and which comprises a polymer controlling the rate of release.

**2.** A preparation in accordance with claim 1, characterized in that the polymer is an ethyl cellulose polymer having a viscosity range of 3-100 mPas (cP) and an ethoxy content of 45-49.5%.

**3.** A preparation in accordance with claim 1, characterized in that the water-insoluble polymeric matrix also contains an oily or wax-like material.

**4.** A preparation in accordance with claim 3, characterized in that the oily or wax-like material is stearic acid, stearyl alcohol, cetyl alcohol, carnauba wax, beeswax, white wax or a glyceride of $C_6$-$C_{18}$-fatty acids.

**5.** A preparation in accordance with claim 4, characterized in that the membrane coating consists of ethyl cellulose or mixtures of ethyl cellulose and hydroxypropyl methylcellulose or hydroxypropyl cellulose.

**6.** A preparation in accordance with claim 5, characterized in that the membrane coating consists of ethyl cellulose.

**7.** A preparation in accordance with claim 5, characterized in that the membrane coating also contains a plasticizer.

**8.** A preparation in accordance with claim 5, characterized in that the plasticizer is triacetin, propylene glycol, polyethylene glycol having a molecular weight of 200 to 1000, dibutyl phthalate, dibutyl sebacate, triethyl citrate, fatty acids or a glyceride of $C_6$-$C_{18}$-fatty acids.

**9.** A preparation in accordance with claim 8, characterized in that the plasticizer is a glyceride of $C_6$-$C_{18}$-fatty acids.

**Revendications**

**1.** Composition pharmaceutique à l'état de comprimés, caractérisée en ce que (1) elle contient un noyau qui constitue de 90 à 95% du poids du comprimé et qui consiste
a) pour 65 à 95% en poids en une substance active soluble dans l'eau et
b) pour 5 à 35% en poids en éthylcellulose et
(2) un revêtement formant membrane, représentant de 5 à 10% du poids du comprimé, et qui consiste en un polymère contrôlant la vitesse de libération.

**2.** Composition selon la revendication 1, caractérisée en ce que le polymère est un polymère d'éthylcellulose à une viscosité dans l'intervalle de 3 à 100 mPa.s et une teneur en groupes éthoxy de 45 à 49,5%.

**3.** Composition selon la revendication 1, caractérisée en ce que la gangue polymère insoluble dans l'eau contient en outre une matière huileuse ou cireuse.

**4.** Composition selon la revendication 3, caractérisée en ce que la matière huileuse ou cireuse est l'acide stéarique, l'alcool stéarylique, l'alcool cétylique, la cire de carnauba, la cire d'abeilles, la cire blanche ou un glycéride d'acides gras en C6-C18.

**5.** Composition selon la revendication 4, caractérisée en ce que le revêtement formant membrane consiste en éthylcellulose ou en un mélange d'éthylcellulose et d'hydroxypropylméthylcellulose ou d'hydroxypropylcellulose.

**6.** Composition selon la revendication 5, caractérisée en ce que le revêtement formant membrane consiste en éthylcellulose.

**7.** Composition selon la revendication 5, caractérisée en ce que le revêtement formant membrane contient en outre un plastifiant.

**8.** Composition selon la revendication 5, caractérisée en ce que le plastifiant est la triacétine, le propylène-glycol, un polyéthylène-glycol de poids moléculaire 200 à 1 000, le phtalate, de dibutyle, le sébaçate de dibutyle, le citrate de triéthyle, un acide gras ou un glycéride d'acides gras en C6-C18.

**9.** Composition selon la revendication 8, caractérisée en ce que le plastifiant est un glycéride d'acides gras en C6-C18.